# EUROPÄISCHE PATENTSCHRIFT

(11) **EP 1 663 119 B1**
(45) Veröffentlichungstag und Bekanntmachung des Hinweises auf die Patenterteilung: **31.03.2010**
(21) Anmeldenummer: 04712574.5
(22) Anmeldetag: 19.02.2004
(51) Int. Cl.: A61K 8/37, A61K 8/46, A61K 8/60, A61Q 5/02, A61Q 19/10

(54) **SCHÄUMBARE KOSMETISCHE REINIGUNGSZUBEREITUNG**
FOAMABLE COSMETIC CLEANSING PREPARATION
PREPARATION DE LAVAGE COSMETIQUE MOUSSANTE

(30) Priorität: 21.02.2003 DE 10307469
(43) Veröffentlichungstag der Anmeldung: 07.06.2006
(73) Patentinhaber: Cognis IP Management GmbH, 40589 Düsseldorf (DE)
(72) Erfinder: DOERSCHNER, Albrecht, 20146 Hamburg (DE); WILLEMS, Elke, 20251 Hamburg (DE)
(86) Internationale Anmeldenummer: PCT/EP2004/050160
(87) Internationale Veröffentlichungsnummer: WO 2004/073666

(56) Entgegenhaltungen:
- EP-A- 1 138 311
- EP-A- 1 174 122
- EP-A- 1 374 845
- WO-A-02/056855
- DE-A- 4 140 474

## Beschreibung

Die vorliegende Erfindung betrifft eine kosmetische Reinigungszubereitung enthaltend anionische und nichtionische Tenside sowie Glycerinmonooleat.

Die Produktion von kosmetischen Reinigungsmitteln zeigt seit Jahren eine steigende Tendenz. Dies ist vor allem auf das zunehmende Gesundheitsbewußtsein und Hygienebedürfnis der Verbraucher zurückzuführen.

Reinigung des menschlichen Körpers bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht abzuwaschen.

Kosmetische Reinigungspräparate sind sogenannte "rinse off" Präparate, welche nach der Anwendung von der Haut abgespült werden. Sie werden in aller Regel in Form eines Schaums mit Wasser auf die zu reinigenden Körperpartien aufgetragen. Basis aller kosmetischen oder dermatologischen Reinigungspräparate sind waschaktive Tenside. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfemung und -lösung, ein leichtes Abspülen und - je nach Wunsch - auch für Schaumregulierung. Damit ist die Grundlage für die Schmutzentfernung lipidhaltiger Verschmutzungen gegeben.

Üblicherweise werden kosmetische Reinigungszubereitungen in zähflüssiger oder fester Form dargereicht und erst bei der Anwendung auf der Haut durch Verreiben mit den Händen aufgeschäumt. Derartige Zubereitungen müssen einen relativ hohen Anteil an gut schäumenden Tensiden (z.B. Seifen) enthalten, da durch das Reiben zweier Hautflächen gegeneinander nur geringe Scherkräfte entstehen, die benötigt werden, um Luft in die Zubereitung einzutragen und diese aufzuschäumen. Solche Zubereitungen haben jedoch den Nachteil, dass sie in der Regel nicht besonders hautverträglich sind und bei empfindlichen Personen zu Hautirritationen führen können.

Um diesem Mangel des Standes der Technik abzuhelfen, wurden Schaumspender (auch Schaumdispenser genannt) entwickelt, mit denen die Reinigungszubereitungen bei der Entnahme aus dem Verpackungsbehältnis aufgeschäumt werden. Durch die hohen Scherkräfte, die beim Durchpressen der Zubereitung durch ein feinmaschiges Netz enstehen, wird Luft in die Reinigungsformulierung eingetragen und diese aufgeschäumt. Dieses Verfahren ist so wirkungsvoll, dass man auch mit schwach schäumenden, hautverträglicheren Tensiden in niedrigen Konzentrationen feinblasige, stabile Schäume erhält. Diese können dann auch zur Reinigung der besonders empfindlichen Gesichtshaut eingesetzt werden.

Der Stand der Technik kennt eine Reihe derartiger Reinigungszubereitungen:
Die Schrift DE 4428823 offenbart Detergensgemische mit verbessertem Schaumvermögen, enthaltend a) bestimmte Alkyl- und Alkenyloligoglykoside mit Alkyl- und/oder Alkenylresten mit 4 bis 22 Kohlenstoffatomen und 1 bis 10 Zuckerresten mit 5 oder 6 Kohlenstoffatomen b) Acylglutamate mit linearen oder verzweigten Acylresten mit 6 bis 22 Kohlenstoffatomen und 0 und/oder 1, 2 oder 3 Doppelbindungen, wobei die Carboxylfunktionen der Glutaminsäure Wasserstoff, ein Alkali- und/oder Erdalkalimetall, Ammonium, Alkylammonium, Alkanolammonium oder Glucammonium tragen können.

Meist werden diesen Zubereitungen geringe Mengen an lipophilen Komponenten zugesetzt, welche die Pflegeeigenschaften der Zubereitungen durch die Rückfettung der Haut verbessern:

Die Schrift DE 10034636 offenbart beispielsweise ein wäßriges, sehr mades, tensidhaltiges Mittel zur Behandlung der Haare oder der Haut, das Ester von Di- oder Tricarbonsäuren mit polyhydroxylierten Substanzen (A) und als zweite Komponente Fettsäurepartialglycaride (B) enthält.

Die Schrift DE 19926526 offenbart eine Verbesserung der Hautverträglichkeit und der Schaumeigenschaften von flüssigen wäßrigen Detergensgemischen, enthaltend 5 bis 40 Gew.-% mindestens eines nichtionischen Tensids, insbesondere C8-C14-Alkylpolyglucosid, vorzugsweise in Kombination mit mindestens einem weiteren anionischen, amphoteren und/oder zwitterionischen Tensid, sowie von 0,25 bis 10 Gew.-%, berechnet auf die Gesamtzusammensetzung des Mittels, mindestens eines Glycerinmono-C8-C18-fettsäureesters, insbesondere Glycerylmonolaurat. Die in dieser Schrift offenbarten Zubereitungen sind zähflüssig und weisen eine Viskosität von 1 bis 10 Pas auf.

Die Schrift DE 10007321 offenbart ein Schaumreinigungsprodukt, bestehend aus einem wäßrigen Reinigungsmittel und einem treibgasfreien Spendebehälter mit Schaumventil, bei dem das wäßrige Reinigungsmittel 5-20 Gew.-% eines oberflächenaktiven Sulfobernsteinsäuremonoestersalzes, 1-10 Gew.-% eines oberflächenaktiven Ampho- oder Betaintensids und 60-90 Gew.-% Wasser enthält. Es eignet sich zur Erzeugung eines besonders feinblasigen und cremigen Reinigungsschaumes mit guter Hautverträglichkeit. Offenbart sind außerdem Schaumreinigungsprodukte, die 1-10 Gew.-% nichtionische Tenside und 0,1-1 Gew.-% eines Fettsäuremonoglycerids einer Fettsäure mit 12-18 C-Atomen enthalten.

Die Schriften DE 10148392 und DE 10148393 offenbaren Pumpschäumer, die Reinigungszubereitungen mit anionischen und nichtionischen Tensiden sowie wasserlöslichen ethoxylierten Fettsäuremonoestem und Fettsäuretriestern enthalten.

Die Schrift WO 02/17876 offenbart schaumbildende Reingungsprodukte, die 0,1 bis 12 Gew.% Tenside , 0,01 bis 3 Gew.% kationisches Polymer, 0,05 bis 1,5 Gew.% eines hydrophoben antibakteriellen Stoffes in einem handbetätigbarem Schaumspender enthalten, die weniger als 0,05 Gew.% eines wasserlöslichen Weichmachers enthalten.

Die Schrift EP 1174122 offenbart eine wässrige Reinigungszubereitung enthaltend ein Schäumendes Tensid und ein glycerintriester.

Die Schrift EP 1 138 311 offenbart eine wässrige Reinigungszubereitung enthaltend Monoglycosid, Acylglutamat und Monoglyceride.

Der Stand der Technik offenbart jedoch keine dünnflüssigen, mit einem Schaumspender gut aufschäumbaren Reinigungszubereitungen, die eine Kombination aus nichtionischen und anionischen Tensiden sowie Glycerinmonooleat enthalten.

Es war daher die Aufgabe der vorliegenden Erfindung, den Mangel des Standes der Technik zu beseitigen und dünnflüssige, mit einem Schaumspender gut aufschäumbare Reinigungszubereitungen, die eine Kombination aus nichtionischen und anionischen Tensiden sowie größere Mengen an lipophilen Komponenten enthalten, zu entwickeln.

Überraschend gelöst wird die Aufgabe durch eine kosmetische und/oder dermatologische Reinigungszubereitung enthaltend eine Stoffkombination aus
a) einem oder mehreren nichtionischen Tensiden gewählt aus der Gruppe Decylglycosid, Laurylglycosid, Kokosglycosid,
b) einem oder mehreren anionischen Tensiden gewählt aus der Gruppe Natriumcocoylglutamat, Natriumlaurylethersulfat, Natriumsarcosinat, Natriummyristylethersulfat,
c) Glycerinmonooleat,
neben gegebenenfalls weiteren kosmetischen und/oder dermatologischen Wirk-, Hilfs- und Zusatzstoffen,
mit einem Wassergehalt von mindestens 75 Gewichts-%, bezogen auf das Gesamtgewicht der Zubereitung und einer Viskosität kleiner 1000 mPas, vorzugsweise kleiner 500 mPas, ganz besonders bevorzugt kleiner 200 mPas, bestimmt mit Hilfe eines Viskosimeters des Typs Viskotester VT 02 der Gesellschaft Haake.

In die erfindungsgemäßen Zubereitungen lassen sich überraschend große Mengen an der hautpflegenden lipophilen Komponente Glycerinmomooleat einarbeiten ohne das ihre Fähigkeit zum Aufschäumen darunter leidet. Dies ist um so überraschender, da lipophile Verbindungen schaumhemmende Eigenschaften aufweisen und in der Technik als "Entschäumer" Verwendung finden. Des weiteren war es überraschend, dass sich Glycerinmonooleat stabil (temperatur-, lager- und langzeitstabil) in die dünnflüssige Zubereitung einarbeiten läßt, ohne dass es zu einer Phasentrennung von wässriger und lipophiler Phase kommt.

Die erfindungsgemäßen Zubereitungen zeichnen sich durch einen hohe Reinigungs- und Hautpflegeleistung aus. Sie bilden besonders cremige, feinblasige und stabile Schäume. Die erfindungsgemäße Zubereitung erzeugt ein überraschend angenehmes Hautgefühl. Auch war es für den Fachmann nicht vorhersehbar, dass die erfindungsgemäßen Zubereitungen transparent erscheinen.

Es ist erfindungsgemäß bevorzugt, wenn als nichtionisches Tensid Decylglycosid eingesetzt wird.

Es ist ferner erfindungsgemäß bevorzugt, wenn als anionisches Tensid Natriumcocoylglutamat eingesetzt wird.

Es ist erfindungsgemäß vorteilhaft, wenn die erfindungsgemäßen Zubereitungen einen Wassergehalt von mindestens 75 Gewichts-%, und besonders bevorzugt einen Wassergehalt von mindestens 85 Gewichts%, jeweils bezogen auf das Gesamtgewicht der Zubereitung aufweisen.

Erfindungsgemäß vorteilhaft weisen die erfindungsgemäßen Zubereitungen eine Viskosität von kleiner als 1000 mPas und bevorzugt eine Viskosität von kleiner 500 mPas und ganz besonders bevorzugt von kleiner 200 mPas auf. Erfindungsgemäß wird die Viskosität dabei nach mit dem Messgerät Viskotester VT02 der Gesellschaft Haake unter den folgenden Rahmenbedingungen (Temperatur 25°C, relative Luftfeuchtigkeit 30 % bis 50%) bestimmt.

Vorteilhaft im Sinne der vorliegenden Erfindung sind Reinigungszubereitungen, die eine Viskosität von mindestens 10 mPas, bevorzugt eine Viskosität von mindestens 50 mPas und besonders bevorzugt eine Viskosität von mindestens 100 mPas aufweisen.

Es ist erfindungsgemäß von Vorteil, wenn ein oder mehrere nichtionische Tenside in einer Gesamtkonzentration von 0,1 bis 10 Gewichts-%, bevorzugt in einer Gesamtkonzentration von 1 bis 5 Gewichts-%, jeweils bezogen auf das Gesamtgewicht der Zubereitung eingesetzt werden.

Es ist erfindungsgemäß von Vorteil, wenn ein oder mehrere anionische Tenside in einer Gesamtkonzentration von 0,1 bis 10 Gewichts-%, bevorzugt in einer Gesamtkonzentration von 1 bis 5 Gewichts-%, jeweils bezogen auf das Gesamtgewicht der Zubereitung eingesetzt werden.

Es ist erfindungsgemäß von Vorteil, wenn Glycerinmonooleat in einer Konzentration von 0,1 bis 5 Gewichts-%, bevorzugt in einer Konzentration von 0,8 bis 2 Gewichts%, jeweils bezogen auf das Gesamtgewicht der Zubereitung eingesetzt wird.

Es ist erfindungsgemäß von Vorteil, wenn das Gewichtsverhältnis von Tensiden (Gesamtmenge aus anionischen und nichtionischen Tensiden) zu Glycerinmonooleat in der erfindungsgemäßen Zubereitung von 1:1 bis 10:1, bevorzugt von 3:1 bis 6:1 beträgt

Auch ist es erfindungsgemäß vorteilhaft, wenn das Gewichtsverhältnis von anionischen zu nichtionischen Tensiden in der erfindungsgemäßen Zubereitung von 0,1:1 bis 10:1, bevorzugt von 0,5:1 bis 4:1 beträgt.

Die erfindungsgemäßen Zubereitungen können erfindungsgemäß vorteilhaft weitere kosmetische oder dermatologische Wirk-, Hilfs- und Zusatzstoffe enthalten.

Beispielweise kann die Zubereitung weitere Tenside enthalten. Diese können vorteilhaft aus der folgenden Liste gewählt werden:

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumoocoylsarkosinat,
4. Taurate, beispielsweise Natriumlauroyltaurat und Natriummethylcocoyltaurat,
5. Acyllactylate, Lauroyllactylat, Caproyllactylat
6. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Arylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl-und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/DEA/MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Vorteilhaft kann die erfindungsgemäße Zubereitung ein oder mehrere dieser zusätzlichen Tenside in einer Konzentration von bis zu 20 Gewichts-%, bezogen auf das Gesamtgewicht der Zubereitung enthalten.

Ferner können Polysorbate als waschaktive Agentien erfindungsgemäß vorteilhaft in die Zubereitung eingearbeitet werden.
Im Sinne der Erfindung vorteilhafte Polysorbate sind dabei das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).

Ganz besonders vorteilhaft sind insbesondere
- Polyoxyethylen(20)sorbitanmonopalmitat (Tween 40, CAS-Nr. 9005-66-7)
- Polyoxyethylen(20)sorbitanmonostearat (Tween 60, CAS-Nr. 9005-67-8).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der Formulierung einzeln oder als Mischung mehrer Polysorbate, eingesetzt.

Die erfindungsgemäße Zubereitung kann in ihrer wässrigen Phase neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Die erfindungsgemäße Zubereitung kann erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Caliumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-Iod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate.

Es ist dabei erfindungsgemäß besonders bevorzugt, wenn als Konservierungsstoffe Iodopropylbutylcarbamate, Parabene (Methyl-, Ethyl-, Propyl- und/oder Butylparaben) und/oder Phenoxyethanol eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung enthalten.

Die erfindungsgemäße Zubereitung enthält vorteilhafter Weise einen oder mehrere Konditionierer. Erfindungsgemäß bevorzugte Konditionierer sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische inhaltsstoffe (INCI) als Polyquaternium benannten Verbindungen dar (insbesondere Polyquaternium-1 bis Polyquaternium-56).

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Fette, Öle, Wachse, Komplexierungs- und Sequestrierungsagentien, Periglanzagentien, Antischuppenwirkstoffe (z.B. Selendisulfid, Zinkpyrithion, Pirocton, Olamin, Climbazol, Octopirox, Polydocanol und deren Kombinatinen), Antitranspirant-Salze (z.B. saure Aluminium- und/oder Aluminium/Zirkoniumsalze wie Aluminiumchlorhydrat und/oder Aluminium/Zirkonium-chlorhydrat), Pflanzenextrakte, Antioxidantien, Vitamine (z.B. Vitamin A, B1-B12, C, D, E, F, H), Wirkstoffe, Peeling-Stoffe (Abrasiva, z.B. Polymerkügelchen oder -pulver aus Polyethylen, Polypropylen etc. anorganischen Oxiden, Silikaten usw.). Ferner können in der Zubereitung Haarglättungsmittel (z.B. Thioglycolate) enthalten sein. Auch Substanzen zur Einstellung des pH-Wertes (z.B. Zitronensäure, Natriumhydroxid) können erfindungsgemäß in der erfindungsgemäßen Zubereitung (auch in Form ihrer Salze) vorhanden sein.

Insbesondere ist es erfindungsgemäß von Vorteil der erfindungsgemäßen Zubereitung Vitamine, Pflanzenextrakte und UV-Lichtschutzfilter zuzusetzen. So ist beispielsweise der Zusatz von Vitamin-Komplexen (z.B. aus γ-Oryzanal und Calciumsalzen wie Calciumpanthotenat, Calciumchlorid, Calciumacetat) erfindungsgemäß vorteilhaft.

Erfindungsgemäß von Vorteil ist es, wenn die erfindungsgemäße Zubereitung einen oder mehrere Lösungsvermittler enthält. Diese können beispielsweise die Einarbeitung von Parfümstoffen unterstützen. Erfindungsgemäß vorteilhaft werden ein oder mehrere Lösungsvermittler in einer Konzentration von 0,1 bis 2 Gewichts-%, bezogen auf das Gesamtgewicht der Zubereitung, eingesetzt. Erfindungsgemäß vorteilhafte Lösungsvermittler sind beispielsweise mit Polyethylen- und/oder Polypropylenketten veretherte Ester aus Glycerin und Fettsäuren, insbesondere hydrierte Fettsäuren. So sind PEG-100 hydriertes Glycerylpalmitat, PEG-200 hydriertes Glycerylpalmitat und PEG-40 hydriertes Rizinusöl erfindungsgemäß bevorzugt.

Die erfindungsgemäße Zubereitung kann vorteilhaft anfeuchtende bzw. feuchthaltende Mittel (sogenannte Moisturizer) enthalten. Vorteilhafte feuchthaltende Mittel im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist

Es ist auch von Vorteil, den erfindungsgemäßen Zubereitungen Komplexbildner zuzusetzen. Vorteilhaft werden die Komplexbildner gewählt aus der Gruppe bestehend aus Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen, Tetrasodium Iminodisuccinate, Trisodium Etylenediamine Disuccinate.

Ein zusätzlicher Gehalt an Antioxidantien ist im allgemeinen bevorzugt Erfindungsgemäß können als günstige Antioxidantien alle für kosmetische und/oder dermatologische Anwendungen geeigneten oder gebräuchlichen Antioxidantien verwendet werden.

Die erfindungsgemäßen Zubereitungen können auch alle nach der Kosmetikverordnung zugelassenen UV-A-, UV-B- und/oder Breitbandfiltersubstanzen enthalten.

Erfindungsgemäß ist auch das Verfahren zur Herstellung einer Reinigungszubereitung das dadurch gekennzeichnet ist, dass zunächst eine Mischung aus nichtionischen Tensiden (insbesondere Kokosglycosid) und Glycerinmonooleat hergestellt wird, die in die wässrige Phase, welche die übrigen Bestandteile der Zubereitung enthält, eingearbeitet wird.

Erfindungsgemäß ist ferner eine Reinigungszubereitung die nach dem erfindungsgemäßen Verfahren hergestellt wird.

Die erfindungsgemäße Zubereitung wird erfindungsgemäß vorteilhaft in einem Schaumspender aufbewahrt und aus diesem heraus in Form eines Schaumes angewendet Es ist erfindungsgemäß vorteilhaft, wenn als Schaumspender ein Pumpschäumer oder Druckgasbehälter (auch Aerosoldose genannt) verwendet wird. Dabei hat es sich als erfindungsgemäß besonders vorteilhaft herausgestellt, wenn als Schaumspender ein Pumpschäumer eingesetzt wird.

Insbesondere ist es erfindungsgemäß, wenn ein solcher mechanischer Schaumspender mittels eines kolbenbetriebenen Pumpsystems die erfindungsgemäße Zubereitung mit hoher Geschwindigkeit durch ein siebartiges Gewebe führt und sie dadurch aufschäumt.

In einer erfindungsgemäß vorteilhaften Ausführungsform verfügt ein solch erfindungsgemäßer mechanischer Schaumspender über einen Spritzwasserschutz.

Die erfindungsgemäßen Reinigungszubereitungen können aber auch vorteilhaft mit einem Treibgas aufgeschäumt werden. Dabei ist es vorteilhaft, das Treibgas in einer Menge von 0,5 bis 30 Gewichts-%, besonders bevorzugt in einer Konzentration von 1 bis 20 Gewichts-% und ganz besonders bevorzugt in einer Konzentration von 3 bis 15 Gewichts-% bezogen auf das Gesamtgewicht der Formulierung einzusetzen.

Die erfindungsgemäß bevorzugten Treibgase sind Propan, Isobutan und n-Butan sowie deren Mischungen. Aber auch Druckluft, Kohlendioxid, Stickstoff, Stickstoffdioxid und Dimethylether sowie Mischungen all dieser Gase sind erfindungsgemäß vorteilhaft zu verwenden. Erfindungsgemäß ganz besonders bevorzugt sind Treibgasmischungen aus Propan und Butan.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Winkung auf die Umwelt oder sonstiger Begleitumstände verzichtet werden sollte, insbesondere Fluorkohlenwasserstoffe und Fluorchlorkohlenwasserstoffe (FCKW). Auch diese Gase wären erfindungsgemäß vorteilhaft einsetzbar.

Erfindungsgemäß vorteilhaft ist die Verwendung der erfindungsgemäßen kosmetischen Zubereitung als Reinigungszubereitung für die Haut.

Die erfindungsgemäße Zubereitung kann aber auch vorteilhaft zur Reinigung der Haare und/oder Nägel verwendet werden.

Erfindungsgemäß ist insbesondere die Verwendung der erfindungsgemäßen Zubereitung zur Reinigung der Gesichtshaut. Bei einer solchen Anwendung ist dabei sowohl die Reinigung der Gesichtshaut von Verunreinigungen wie beispielsweise Talg, Sebum, Schweiß, abgestorbene Hautpartikel erfindungsgemäß, als auch die Verwendung zur Entfernung dekorativer Kosmetika von der Haut (sog. Abschminken). Insbesondere die Entfernung von Lidschatten, Wimpemtusche (Maskara), Foundation, Lippenstift, Puder etc. ist erfindungsgemäß.

Ferner eignen sich die erfindungsgemäßen Produkte hervorragend zur Reinigung von Gegenständen des täglichen Lebens (z.B. Geschirr, Tisch- und Schrankflächen, Autos, Kleidung).

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

| | **1** | **2** | **3** | **4** | **5** | **6** |
|---|---|---|---|---|---|---|
| Natrium Cocoylglutamat | 2,5 | - | - | - | 3 | - |
| Natrium Laurylethersulfat | - | 3,5 | - | - | - | 4 |
| Natrium Lauroylsarkosinat | - | - | 5 | - | - | - |
| Natrium Myristylethersulfat | - | - | - | 4,5 | - | - |
| Decylglucosid | 3 | 4 | - | - | - | - |
| Laurylglucosid | - | - | 2 | 3 | - | - |
| Coco Glucoside | - | - | - | - | 4 | 3 |
| Glyceryl Oleate | 0,8 | 2 | 1 | 1 | 1 | 1 |
| PEG-200 hydriertes Glycerylpalmitat | 0,5 | - | - | - | 0,5 | - |
| PEG-40 hydriertes Rizinusöl | 0,1 | - | 0,1 | 0,1 | 0,1 | 0,1 |
| PEG-100 hydriertes Glycorylpalmitat | - | - | - | 0,5 | - | 0,5 |
| Natriumbenzoat | 0,5 | 0,5 | - | 0,5 | 0,5 | - |
| Natriumsalicylat | - | 0,2 | - | 0,2 | 0,2 | - |
| Methyldibromoglutaronitril | - | - | 0,04 | - | - | 0,04 |
| Phenoxyethanol | - | - | 0,16 | - | - | 0,16 |
| Jojobaöl (Buxus Chinensis) | 0,1 | - | - | - | - | - |
| Polyquaternium-10 | - | 0,1 | - | 0,2 | - | - |
| Citronensäure | q.s. | q.s. | q.s. | q.s. | q.s. | q.s. |
| Parfüm | q.s. | q.s. | qs. | q.s. | q.s. | q.s. |
| Wasser | ad 100 | ad 100 | ad 100 | ad 100 | ad 100 | ad 100 |

## Patentansprüche

1. Kosmetische und/oder dermatologische Reinigungszubereitung enthaltend eine Stoffkombination aus
a) einem oder mehreren nichtionischen Tensiden gewählt aus der Gruppe Decylglycosid, Laurylglycosid, Kokosglycosid,
b) einem oder mehreren anionischen Tensiden gewählt aus der Gruppe Natriumcocoylglulamat, Natriumlaurylethersulfat, Natriumsarcosinat, Natriummyristylethersulfat,
c) Glycerinmonooleat,
neben gegebenenfalls weiteren kosmetischen und/oder dermatologischen Wirk-, Hilfs-und Zusatzstoffen,
mit einem Wassergehalt von mindestens 75 Gewichts-%, bezogen auf das Gesamigewicht der Zubereitung und einer Viskosität von kleiner 1000 mPas.

2. Reiningungszubereitung nach Anspruch 1, **dadurch gekennzeichnet, dass** sie
a) ein oder mehrere nichtionische Tenside in einer Gesamtkonzentration von 0.1 bis 10 gew.%, bevorzugt von 1 bis 5 Gewichts-%,
bezogen auf des Gesamtgewicht der Zubereitung, enthält.

3. Reinigungszubereitung nach Anspruch 1, **dadurch gekennzeichnet, dass** sie
b) ein oder mehrere anionische Tenside in einer Gesamtkonzentration von 0.1 bis 10 gew.%, bevorzugt von 1 bis 5 Gewichts-%,
bezogen auf das Gesamtgewicht der Zubereitung, enthält.

4. Reinigungszubereitung nach Anspruch 1, **dadurch gekennzeichnet, dass** sie
c) Glycerinmonooleat in einer Konzentration von 0.1 bis 5 gew.%, bevorzugt von 0,8 bis 2 Gewichts-%, bezogen auf das Gesamtgewicht der Zubereitung, enthält.

5. Reinigungszubereitung nach Anspruch 1, **dadurch gekennzeichnet, dass** sie
a) ein oder mehrere nichtionische Tenside in einer Gesamtkonzentration von 1 bis 5 Gewichts-%,
b) ein oder mehrere anionische Tenside in einer Gesamtkonzentration von 1 bis 5 Gewichts-%,
c) Glycerinmonooleat in einer Konzentration von 0,8 bis 2 Gewichts-%, jewells bezogen auf das Gesamtgewicht der Zubereitung, enthält.

6. Reinigungszubereitung nach einem der Ansprüche 1 bis 4, **dadurch gekennzeichnet, dass** das Gewichtsverhältnis von anionischen und nichtionischen Tensiden zu Glycerinmonooleat von 1:1 bis 10:1, bevorzugt von 3:1 bis 6:1 beträgt.

7. Reinigungszubereitung nach einem der Ansprüche 1 bis 4, **dadurch gekennzeichnet, dass**, das Gewichtsverhältnis von anionischen zu nichtionischen Tensiden von 0,1:1 bis 10:1, bevorzugt von 0,5:1 bis 4:1 beträgt.

8. Reinigungszubereitung nach einem der Ansprüche 1 oder 5, **dadurch gekennzeichnet, dass** das Gewinchtsverhältnis von anionischen und nichtionischen Tensiden zu Glycerinmonooleat von 3:1 bis 6:1 beträgt.

9. Reinigungszubereitung nach einem der Ansprüche 1 oder 5 oder 6, **dadurch gekennzeichnet, dass** das Gewinchtsverhältnis von anionischen zu nichtionischen Tensiden von 0,5:1 bis 4:1 beträgt.

10. Verfahren zur Herstellung einer Reinigungszubereitung nach einem der Ansprüche 1 oder 5 oder 8, oder 9 **dadurch gekennzeichnet, dass** zunächst eine Mischung aus nichtionischen Tensiden und Glycerinmonooleat hergestellt wird, die in die wässrige Phase, welche die übrigen Bestandteile der enthält, eingearbeitet wird.

11. Reinigungsprodukt aus einem Schaumspender und einer Reiningungszubereitung nach einem der Ansprüche 1 oder 5 oder 8 oder 9.

12. Reinigungsprodukt nach Anspruch 11, **dadurch gekennzeichnet, dass** es sich bei dem Schaumspender um einen Pumpschäumer mit mechanischem Pumpsystem handelt.

## Claims

1. Cosmetic and/or dermatological cleansing preparation comprising a material combination of
a) one or more nonionic surfactants selected from the group consisting of decyl glycoside, lauryl glycoside, cocoa glycoside,
b) one or more anionic surfactants selected from the group consisting of sodium cocoylglutamate, sodium lauryl ether sulphate, sodium sarcosinate, sodium myristyl ether sulphate,
c) glycerol monooleate,
as well as optionally further cosmetic and/or dermatological active ingredients, auxiliaries and additives,
with a water content of at least 75% by weight, based on the total weight of the preparation, and a viscosity of less than 1000 mPas.

2. Cleansing preparation according to Claim 1, **characterized in that** it comprises
a) one or more nonionic surfactants in a total concentration of from 0.1 to 10% by weight, preferably of from 1 to 5% by weight, based on the total weight of the preparation.

3. Cleansing preparation according to Claim 1, **characterized in that** it comprises
b) one or more anionic surfactants in a total concentration of from 0.1 to 10% by weight, preferably of from 1 to 5% by weight, based on the total weight of the preparation.

4. Cleansing preparation according to Claim 1, **characterized in that** it comprises
c) glycerol monooleate in a concentration of from 0.1 to 5% by weight, preferably of from 0.8 to 2% by weight, based on the total weight of the preparation.

5. Cleansing preparation according to Claim 1, **characterized in that** it comprises
a) one or more nonionic surfactants in a total concentration of from 1 to 5% by weight,
b) one or more anionic surfactants in a total concentration of from 1 to 5% by weight,
c) glycerol monooleate in a concentration of from 0.8 to 2% by weight, in each case based on the total weight of the preparation.

6. Cleansing preparation according to one of Claims 1 to 4, **characterized in that** the weight ratio of anionic and nonionic surfactants to glycerol monooleate is from 1:1 to 10:1, preferably from 3:1 to 6:1.

7. Cleansing preparation according to one of Claims 1 to 4, **characterized in that** the weight ratio of anionic to nonionic surfactants is from 0.1:1 to 10:1, preferably from 0.5:1 to 4:1.

8. Cleansing preparation according to one of Claims 1 or 5, **characterized in that** the weight ratio of anionic and nonionic surfactants to glycerol monooleate is from 3:1 to 6:1.

9. Cleansing preparation according to one of Claims 1 or 5 or 6, **characterized in that** the weight ratio of anionic to nonionic surfactants is from 0.5:1 to 4:1.

10. Process for preparing a cleansing preparation according to one of Claims 1 or 5 or 8 or 9, **characterized in that** initially a mixture of nonionic surfactants and glycerol monooleate is prepared, which is incorporated into the aqueous phase which comprises the other constituents of the preparation.

11. Cleansing product composed of a foam dispenser and a cleansing preparation according to one of Claims 1 or 5 or 8 or 9.

12. Cleansing product according to Claim 11, **characterized in that** the foam dispenser is a pump foamer with a mechanical pump system.

## Revendications

1. Préparation nettoyante cosmétique et/ou dermatologique, contenant une combinaison de substances constituée par
a) un ou plusieurs agents tensioactifs non ioniques choisis dans le groupe formé par le décylglycoside, le laurylglycoside, le cocoglycoside,
b) un ou plusieurs agents tensioactifs anioniques choisis dans le groupe formé par le cocoylglutamate de sodium, le lauryléthersulfate de sodium, le sarcosinate de sodium, le myristyléthersulfate de sodium,
c) le monooléate de glycérol,
outre le cas échéant d'autres substances actives, adjuvants et additifs cosmétiques et/ou dermatologiques,
présentant une teneur en eau d'au moins 75% en poids, par rapport au poids total de la préparation et une viscosité inférieure à 1000 mPa.s.

2. Préparation nettoyante selon la revendication 1, **caractérisée en ce qu'**elle contient
a) un ou plusieurs agents tensioactifs non ioniques en une concentration totale de 0,1 à 10% en poids, de préférence de 1 à 5% en poids,
par rapport au poids total de la composition.

3. Préparation nettoyante selon la revendication 1, **caractérisée en ce qu'**elle contient
b) un ou plusieurs agents tensioactifs anioniques en une concentration totale de 0,1 à 10% en poids, de préférence de 1 à 5% en poids,
par rapport au poids total de la composition.

4. Préparation nettoyante selon la revendication 1, **caractérisée en ce qu'**elle contient
c) du monooléate de glycérol en une concentration de 0,1 à 5% en poids, de préférence de 0,8 à 2% en poids,
par rapport au poids total de la composition.

5. Préparation nettoyante selon la revendication 1, **caractérisée en ce qu'**elle contient
a) un ou plusieurs agents tensioactifs non ioniques en une concentration totale de 1 à 5% en poids,
b) un ou plusieurs agents tensioactifs anioniques en une concentration totale de 1 à 5% en poids,
c) du monooléate de glycérol en une concentration de 0,8 à 2% en poids,
à chaque fois par rapport au poids total de la composition.

6. Préparation nettoyante selon l'une quelconque des revendications 1 à 4, **caractérisée en ce que** le rapport pondéral des agents tensioactifs anioniques et non ioniques au monooléate de glycérol est de 1:1 à 10:1, de préférence de 3:1 à 6:1.

7. Préparation nettoyante selon l'une quelconque des revendications 1 à 4, **caractérisée en ce que** le rapport pondéral des agents tensioactifs anioniques aux agents tensioactifs non ioniques est de 0,1:1 à 10:1, de préférence de 0,5:1 à 4:1.

8. Préparation nettoyante selon l'une quelconque des revendications 1 ou 5, **caractérisée en ce que** le rapport pondéral des agents tensioactifs anioniques et non ioniques au monooléate de glycérol est de 3:1 à 6:1.

9. Préparation nettoyante selon l'une quelconque des revendications 1 ou 5 ou 6, **caractérisée en ce que** le rapport pondéral des agents tensioactifs anioniques aux agents tensioactifs non ioniques est de 0,5:1 à 4:1.

10. Procédé pour la confection d'une préparation nettoyante selon l'une quelconque des revendications 1 ou 5 ou 8 ou 9, **caractérisé en ce qu'**on prépare d'abord un mélange d'agents tensioactifs non ioniques et de monooléate de glycérol, qui est incorporé dans la phase aqueuse qui contient les autres constituants de la composition.

11. Produit nettoyant constitué par un distributeur de mousse et une préparation nettoyante selon l'une quelconque des revendications 1 ou 5 ou 8 ou 9.

12. Produit nettoyant selon la revendication 11, **caractérisé en ce qu'**il s'agit, pour le distributeur de mousse, d'un dispositif de moussage à pompe avec un système à pompe mécanique.
